# EUROPÄISCHE PATENTANMELDUNG

(11) **EP 0 806 406 A1**
(43) Veröffentlichungstag der Anmeldung: **12.11.1997**
(21) Anmeldenummer: 97100725.7
(22) Anmeldetag: 17.01.1997
(51) Int. Cl.: C07C 67/54, C07C 69/82

(54) **Verfahren zur Trennung von Rohester im DMT-Prozess**

(30) Priorität: 07.05.1996 DE 19618152
(71) Anmelder: HÜLS AKTIENGESELLSCHAFT, 45764 Marl (DE)
(72) Erfinder: Dahl. Hans Detlef, Dr., 45768 Marl (DE); Lenz, Udo, 45657 Recklinghausen (DE)

(57) **Zusammenfassung**

Die vorliegende Erfindung betrifft ein Verfahren zur Trennung von sogenanntem Rohester, der im wesentlichen durch die katalysierte Oxidation von para-Xylol (p-X) und para-Toluylsäuremethylester (p-TE) und die nachfolgende Veresterung der Reaktionsprodukte aus der Oxidation mit Methanol bei der Herstellung von Dimethylterephthalat (DMT) erhalten wird, in eine p-TE-reiche Fraktion, eine Roh-DMT-Fraktion und eine schwersiedende Rückstandsfraktion, wobei man die Trennung des Rohesters in einer Trenneinheit durchführt, die eine Trennwandkolonne oder eine Destillationskolonne mit einer Vorkolonne oder eine Destillationskolonne mit einer Nachkolonne enthält.

Ferner betrifft die Erfindung ein Verfahren zur Herstellung von Dimethylterephthalat (DMT), wobei man die Trennung des Rohesters in einer Trenneinheit durchführt, die mit einer Trennwandkolonne oder einer Destillationskolonne mit einer Vorkolonne oder einer Destillationskolonne mit einer Nachkolonne ausgestattet ist.

## Beschreibung

Die Erfindung betrifft ein Verfahren zur Trennung von sogenanntem Rohester, der im wesentlichen durch die katalysierte Oxidation von para-Xylol (p-X) und para-Toluylsäuremethylester (p-TE) und die nachfolgende Veresterung der Reaktionsprodukte aus der Oxidation mit Methanol bei der Herstellung von Dimethylterephthalat (DMT) erhalten wird, in eine p-TE-reiche Fraktion, eine Roh-DMT-Fraktion und eine schwersiedende Rückstandsfraktion sowie das damit verbundene Verfahren zur Herstellung von Dimethylterephthalat (DMT).

Es ist bekannt, daß der heutige Witten-DMT-Prozeß im wesentlichen die Verfahrensschritte
- Oxidation von para-Xylol (p-X) und para-Toluylsäuremethylester (p-TE), in der Regel mit nachgeschalteter Abgasreinigung,
- Veresterung der Reaktionsprodukte aus der Oxidation mit Methanol,
- Trennung des entstandenen, sogenannten Rohesters (DMT-roh) in eine p-TE-reiche Fraktion, die üblicherweise in die Oxidation zurückgeführt wird, eine Roh-DMT-Fraktion, die im allgemeinen mehr als 85 Gew.-% DMT enthalt, und eine schwersiedende Rückstandsfraktion, gegebenenfalls deren Aufarbeitung, beispielsweise durch eine nachgeschaltete Methanolyse oder Thermolyse, und eine nachfolgende Rückgewinnung des Katalysators,
- Reinigung der Roh-DMT-Fraktion, beispielsweise durch Waschen, Umkristallisieren und Reindestillation,
beinhaltet ("Terephthalsäuredimethylester", Ullmann Bd. 22, 4. Auflage, S. 529 - 533; EP 0 464 046 B1; DE-OS 40 26 733). Es ist auch möglich aus DMT, d. h. aus besonders DMT-reichen Fraktionen bzw. DMT-reinst, durch eine gezielte Hydrolyse Terephthalsäure entsprechender Qualität herzustellen.

Die Oxidation eines Genlisches aus para-Xylol (p-X) und para-Toluylsäuremethylester (p-TE oder pT-Ester) wird im allgemeinen mit Luftsauerstoff in Gegenwart eines Schwermetallkatalysators (DE-PS 20 10 137) bei einer Temperatur von etwa 140 bis 180 °C und einem Druck von etwa 4 bis 8 bar abs. in flüssiger Phase durchgeführt. Aus der Oxidationsstufe resultiert ein Reaktionsgemisch, das überwiegend Monomethylterephthalat (MMT), p-Toluylsäure (p-TA) und Terephthalsäure (TA) gelöst bzw. suspendiert in p-TE enthält und mit Methanol bei einer Temperatur von etwa 250 bis 280 °C und einem Druck von 20 bis 25 bar abs. verestert wird. In einer Trenneinheit, nachfolgend auch Rohester-Destillation genannt, wird nun der erhaltene Rohester destillativ in eine p-TE-Fraktion, eine Roh-DMT-Fraktion und eine schwersiedende, Katalysator-haltige Rückstandsfraktion aufgetrennt. Die p-TE-Fraktion wird in die Oxidation zurückgeführt und die Roh-DMT-Fraktion über nachfolgende Reinigungsstufen in die gewünschte Produktqualität überführt. Die aus der Rohester-Destillation stammende Rückstandsfraktion kann einer Methanolyse oder einer Thermolyse unterzogen werden, die dabei anfallenden Nutzprodukte werden in den Prozeß zurückgeführt.

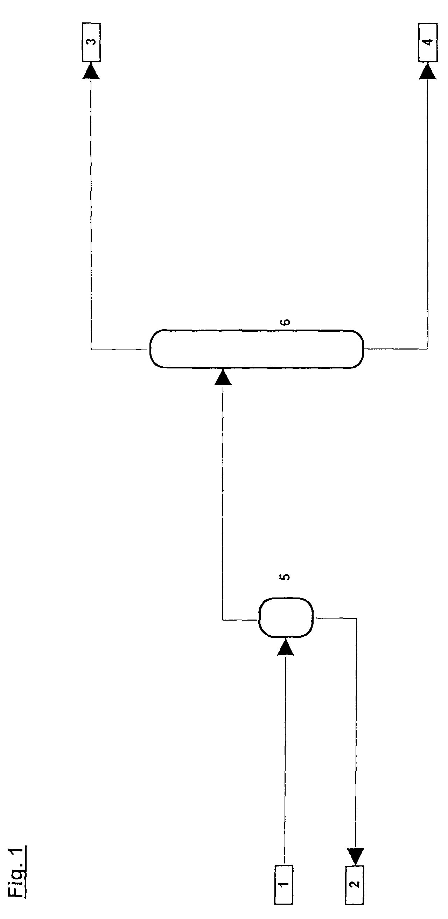

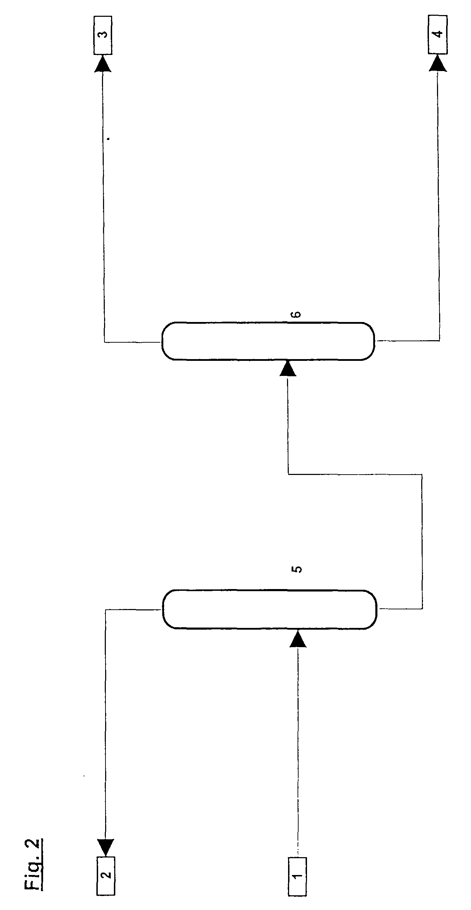

Beim Witten-DMT-Verfahren sind für die Durchführung der Rohester-Destillation üblicherweise das Flashkammersystem, vgl. Fig. 1, sowie das 2-Kolonnensystem, vgl. Fig. 2, im Einsatz. Ein 3-Kolonnensystem ist ebenfalls bekannt (DE-OS 30 11 858). In diesen Systemen ist es jedoch von Nachteil, daß neben der p-TE-reichen Fraktion auch die Roh-DMT-Fraktion über Kopf getrieben werden muß und dadurch ein hoher Energiebedarf entsteht. Außerdem werden in den zuvor genannten Systemen, begünstigt durch hohe Verweilzeiten in den Kolonnensümpfen, deutliche Mengen unerwünschter Nebenprodukte gebildet, die die Produktionsausbeute erheblich mindern. Ferner ist das Flashkammer- und das 2- bzw. 3-Kolonnen-System auch ein wesentlicher Kostenfaktor für den Aufbau, den Betrieb und Erhalt einer DMT-Anlage.

Der Erfindung lag daher die Aufgabe zugrunde, ein Verfahren zur Trennung des Rohesters im DMT-Prozeß bereitzustellen, das es ermöglicht, den Energiebedarf im Prozeß zu verringern und so auch die Wirtschaftlichkeit weiter zu verbessern.

Die gestellte Aufgabe wird erfindungsgemäß entsprechend den Angaben der Patentansprüche gelöst.

Es wurde nun gefunden, daß im DMT-Prozeß deutliche Mengen an Energie eingespart werden können, wenn man die Trennung des Rohesters in einer Trenneinheit durchführt, die eine Destillationskolonne mit Trennwand, kurz auch Trennwandkolonne genannt, oder eine Destillationskolonne mit einer Vorkolonne oder einer Destillationskolonne mit einer Nachkolonne enthält.

Vom Grundsatz her sind Kolonnensysteme mit Destillationskolonnen, die eine Trennwand enthalten, und Kolonnen mit beigeschalteten Hilfskolonnen bekannt und ihre allgemeine Funktionsweise beschrieben:
ECN 2-8 Oktober 1995, S. 26, "BASF distils energy savings"; Chem. Eng. Res. Des. (1993) 71(3), S. 307, "The control of dividing wall column"; US 2,471,134; Chem. Eng. Res. Des., Part A: Trans IChemE, Sep. 1993, S. 639-644, "Heat transfer across the wall of dividing wall"; Chem. Eng. Res. Des., Part A: Trans IChemE, März 1992, S. 118-132, "The Design and optimisation of fully thermally coupled distillation columns".

Der besondere Vorteil beim vorliegenden Verfahren resultiert u. a. daraus, daß die Roh-DMT-Fraktion im Seitenabzug abgeführt werden kann und nicht mehr über Kopf getrieben werden muß. Dadurch können gegenüber den bekannten Verfahren zur Rohester-Destillation erhebliche Energieeinsparungen bei ansonsten unveränderter Roh-DMT-Qualität erzielt werden. Darüber hinaus vermindert eine unter vergleichsweise produktschonenden Bedingungen erreichbare Reduzierung der Verweilzeit von Wertprodukt im heißen Kolonnensumpf die ausbeuteschädliche Bildung unerwünschter Nebenprodukte.

Gegenstand der vorliegenden Erfindung ist somit ein Verfahren zur Trennung von sogenanntem Rohester, der im wesentlichen durch die katalysierte Oxidation von para-Xylol (p-X) und para-Toluylsäuremethylester (p-TE) und die nachfolgende Veresterung der Reaktionsprodukte aus der Oxidation mit Methanol bei der Herstellung von Dimethylterephthalat (DMT) erhalten wird, in eine p-TE-reiche Fraktion, eine Roh-DMT-Fraktion und eine schwersiedende Rückstandsfraktion, das dadurch gekennzeichnet ist, daß man die Trennung des Rohesters in einer Trenneinheit, die eine Trennwandkolonne oder eine Destillationskolonne mit einer Vorkolonne oder eine Destillationskolonne mit einer Nachkolonne enthält, durchführt.

Beim erfindungsgemäßen Verfahren führt man die Trennung des Rohesters im allgemeinen bei einer Temperatur im Bereich von 100 bis 280 °C, vorzugsweise von 130 bis 260 °C, und Drücken im Bereich von 50 bis 500 mbar abs., durch.

Um eine besonders gute Trennwirkung der erfindungsgemäßen Trenneinheiten bei gleichzeitig möglichst geringem Druckanstieg über das System zu erzielen, kann man in die Trennwandkolonne sowie in die Destillationskolonne mit Vorkolonne oder Nachkolonne und/oder in die Vorkolonne oder in die Nachkolonne vorzugsweise sogenannte Strukturpackungen einbauen.

Bevorzugt führt man beim erfindungsgemäßen Verfahren die Trennung des Rohesters in einem 1-Kolonnensystem durch; hier sind auch solche Trenneinheiten einzubeziehen, die geeigneterweise mit einer Destillationskolonne mit beigeschalteter Vor- oder Nachkolonne ausgestattet sind.

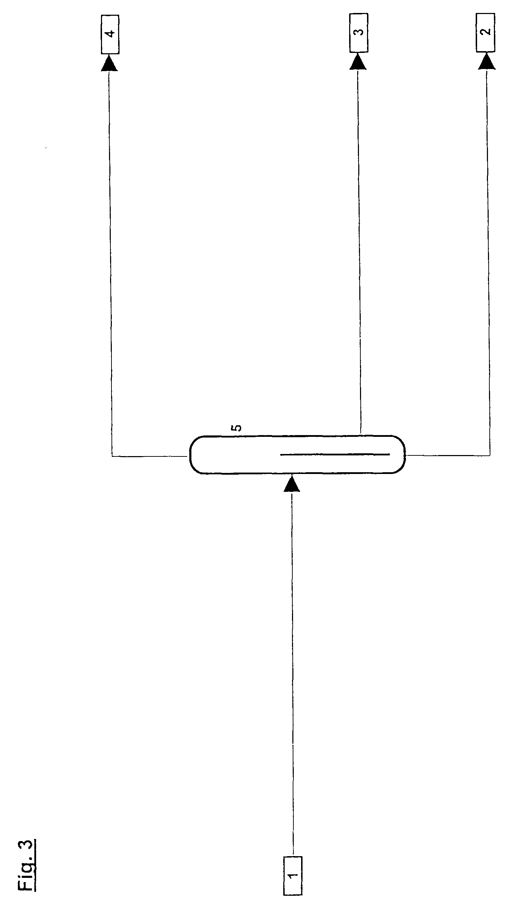

Der Figur 3 ist das Blockschema einer bevorzugten Ausführungsform einer erfindungsgemäßen Trenneinheit für die Trennung des Rohesters im DMT-Prozeß zu entnehmen, wobei diese als Trennwandkolonne mit Seitenabzug ausgeführt ist. Geeigneterweise wird zunächst der Rohester der Trennwandkolonne seitlich der Trennwand zugegeben. Am Kopf der Trennwandkolonne kann die leichter als DMT siedende p-TE-Fraktion abgezogen und in die Oxidation zurückgeführt werden. Geeigneterweise verhindert die Trennwand in der Trennwandkolonne, daß schwersiedende Bestandteile der Rückstandsfraktion sowie Schwermetallkatalysator in die Roh-DMT-Fraktion gelangen. Die Roh-DMT-Fraktion verläßt die Trennwandkolonne in der Regel über einen Seitenabzug, vorzugsweise in Höhe der Trennwand. Am Sumpf der Trennwandkolonne wird im allgemeinen die schwersiedende Rückstandsfraktion abgeführt.

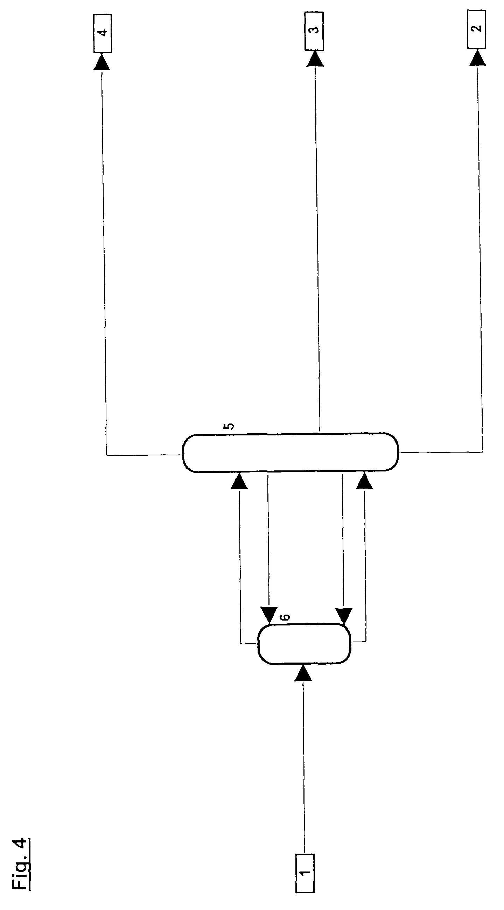

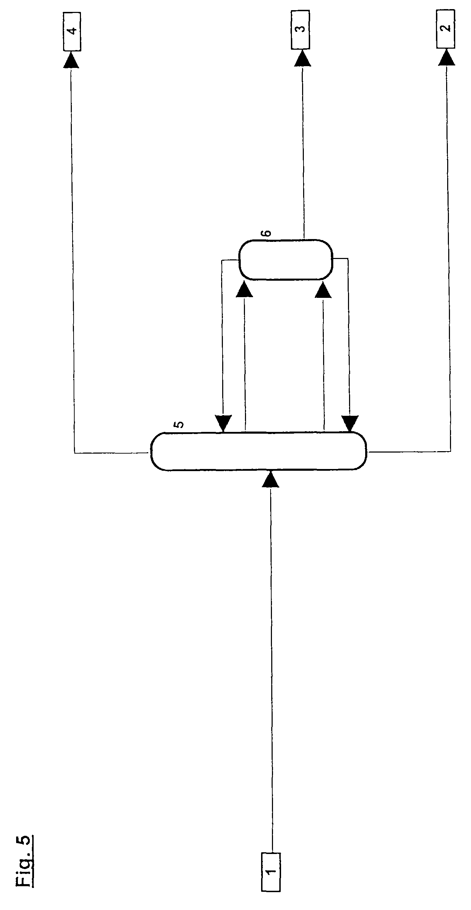

Durch das geeignete Vorschalten oder Nachschalten einer Hilfskolonne, auch Vor- oder Nachkolonne genannt, zu einer Destillationskolonne kann erfindungsgemäß ebenso verhindert werden, daß bei der Trennung des Rohesters bereits zuvor erwähnte Bestandteile der Rückstandsfraktion in die Roh-DMT-Fraktion gelangen, vgl. hierzu Fig. 4 und 5.

Figur 4 zeigt das Blockschema einer anderen bevorzugten Ausführungsform einer erfindungsgemäßen Trenneinheit für die Trennung des Rohesters im DMT-Prozeß, wobei diese als Destillationskolonne mit Seitenabzug für die Roh-DMT-Fraktion und beigeschalteter Vorkolonne ausgeführt ist. Geeigneterweise wird der Rohester zunächst der Vorkolonne seitlich zugeführt.

Aus Figur 5 ist das Blockschema einer weiteren bevorzugten Ausführungsform einer erfindungsgemäßen Trenneinheit für die Trennung des Rohesters im DMT-Prozeß zu entnehmen, wobei diese als Destillationskolonne mit beigeschalteter Nachkolonne einschließlich Seitenabzug für die Roh-DMT-Fraktion ausgeführt ist.

Im allgemeinen verarbeitet man die bei der Trennung des Rohesters anfallende Roh-DMT-Fraktion weiter, vorzugsweise zu Dimethylterephthalat mit höherer Reinheit, z. B. DMT-reinst, sowie zu Terephthalsäure (TA) entsprechender Qualität, z. B. PTA oder PTA-p, die geeigneterweise durch Hydrolyse der jeweiligen DMT-Qualität erhältlich ist. Zur Weiterverarbeitung der Roh-DMT-Fraktion kann, wie eingangs bereits beschrieben, verfahren werden.

Gegenstand der vorliegenden Erfindung ist daher auch ein Verfahren zur Herstellung von Dimethylterephthalat (DMT), das im wesentlichen folgende Verfahrensschritte beinhaltet,
- Oxidation von para-Xylol (p-X) und para-Toluylsäuremethylester (p-TE),
- Veresterung der Reaktionsprodukte aus der Oxidation mit Methanol,
- Trennung des entstandenen, sogenannten Rohesters in eine p-TE-reiche Fraktion, eine Roh-DMT-Fraktion und eine schwersiedende Rückstandsfraktion,
- Reinigung der Roh-DMT-Fraktion,
nach mindestens einem der Ansprüche 1 bis 6, das dadurch gekennzeichnet ist, daß man die Trennung des Rohesters in einer Trenneinheit durchführt, die mit einer Trennwandkolonne oder einer Destillationskolonne mit einer Vorkolonne oder einer Destillationskolonne mit einer Nachkolonne ausgestattet ist.

Der Vorteil des erfindungsgemäßen Verfahrens besteht auch darin, daß der Investitionsaufwand für erfindungsgemäße Trenneinheiten im allgemeinen deutlich geringer ist als für vergleichbare herkömmliche Systeme.

### Legende zu Fig. 1

Figur 1 zeigt ein Blockschema einer Rohester-Destillation im DMT-Prozeß, das als Flashkammersystem bezeichnet wird:

### - Stoffströme -

**1** Rohester aus der Veresterung
**2** Hochsiedende, Katalysator-haltige Rückstandsfraktion zur Aufarbeitung
**3** Rückführung der p-TE-Fraktion in die Oxidation
**4** Roh-DMT zur weiteren Reinigung

### - Anlagenteile -

**5** Flashkammer
**6** Rohester-Destillationskolonne

### Legende zu Fig. 2

Figur 2 zeigt ein Blockschema einer Rohester-Destillation im DMT-Prozeß, die als Zwei-Kolonnen-System bezeichnet wird:

### - Stoffströme -

**1** Rohester aus der Veresterung
**2** Rückführung der p-TE-Fraktion in die Oxidation
**3** Roh-DMT-Fraktion zur weiteren Reinigung
**4** Hochsiedende, Katalysator-haltige Rückstandsfraktion zur Aufarbeitung

### - Anlagenteile -

**5** p-TE-Destillationskolonne
**6** Roh-DMT-Destillationskolonne

### Legende zu Fig. 3

Figur 3 zeigt das Blockschema einer bevorzugten Ausführungsform einer erfindungsgemäßen Trenneinheit für den Rohester im DMT-Prozeß, die als Trennwanddestillationskolonne mit Seitenabzug ausgeführt ist:

### - Stoffströme -

**1** Rohester aus der Veresterung
**2** Hochsiedende, Katalysator-haltige Rückstandsfraktion zur Aufarbeitung
**3** Seitenabzug der Roh-DMT-Fraktion zur weiteren Reinigung
**4** Rückführung der p-TE-Fraktion in die Oxidation

### - Anlagenteile -

**5** Trennwanddestillationskolonne

### Legende zu Fig. 4

Figur 4 zeigt das Blockschema einer bevorzugten Ausführungsform einer erfindungsgemäßen Trenneinheit für den Rohester im DMT-Prozeß, die als Destillationskolonne mit Seitenabzug und beigeschalteter Vorkolonne ausgeführt ist:

### - Stoffströme -

**1** Rohester aus der Veresterung
**2** Hochsiedende, Katalysator-haltige Rückstandsfraktion zur Aufarbeitung
**3** Seitenabzug der Roh-DMT-Fraktion zur weiteren Reinigung
**4** Rückführung der p-TE-Fraktion in die Oxidation

### - Anlagenteile -

**5** Rohester-Destillationskolonne
**6** Vorkolonne

### Legende zu Fig. 5

Figur 5 zeigt das Blockschema einer bevorzugten Ausführungsform einer erfindungsgemäßen Trenneinheit für den Rohester im DMT-Prozeß, die als Destillationskolonne und beigeschalteter Nachkolonne mit Seitenabzug ausgeführt ist:

### - Stoffströme -

**1** Rohester aus der Veresterung
**2** Hochsiedende, Katalysator-haltige Rückstandsfraktion zur Aufarbeitung
**3** Seitenabzug der Roh-DMT-Fraktion zur weiteren Reinigung
**4** Rückführung der p-TE-Fraktion in die Oxidation

### - Anlagenteile -

**5** Rohester-Destillationskolonne
**6** Nachkolonne

### Legende der Abkürzungen:

- p-X: : para-Xylol
- p-TA: : para-Toluylsäure
- p-TE: : para-Toluylsäuremethylester (pT-Ester)
- BME: : Benzoesäuremethylester
- HM-BME: : Hydroxymethylbenzoesäuremethylester
- MM-BME: : Methoxymethylbenzoesäuremethylester
- DMT: : Dimethylterephthalat
- DMT-roh: = Rohester (DMT-Rohesterstrom nach der Veresterung)
- Roh-DMT: : Dimethylterephthalatfraktion nach der Rohester-Destillation
- DMT-reinst: : Dimethylterephthalat-reinst (hochreines DMT-Zwischen- oder Endprodukt)
- DMO: : Dimethylorthopthalsäure
- DMI: : Dimethylisophthalsäure
- DMP: : Dimethylphthalate = Isomerengemisch aus DMT, DMO und DMI
- MMT: : Monomethylterephthalat (Terephthalsäuremonomethylester)
- TA: : Terephthalsäure
- MTA: : mittelreine Terephthalsäure
- PTA: : Terephthalsäure hoher Reinheit
- PTA-p: : Terephthalsäure sehr hoher, d. h. höchster Reinheit (Gehalt an MMT und p-TA von zusammen < 50 Gew.-ppm)
- TAS: : Terephthalaldehydsäure (4-CBA)
- TAE: : Terephthalaldehydsäuremethylester

## Patentansprüche

1. Verfahren zur Trennung von sogenanntem Rohester,
der im wesentlichen durch die katalysierte Oxidation von para-Xylol (p-X) und para-Toluylsäuremethylester (p-TE) und die nachfolgende Veresterung der Reaktionsprodukte aus der Oxidation mit Methanol bei der Herstellung von Dimethylterephthalat (DMT) erhalten wird,
in eine p-TE-reiche Fraktion, eine Roh-DMT-Fraktion und eine schwersiedende Rückstandsfraktion,
dadurch gekennzeichnet,
daß man die Trennung des Rohesters in einer Trenneinheit, die eine Trennwandkolonne oder eine Destillationskolonne mit einer Vorkolonne oder eine Destillationskolonne mit einer Nachkolonne enthält, durchführt.

2. Verfahren nach Anspruch 1,
dadurch gekennzeichnet,
daß man die Trennung des Rohesters bei einer Temperatur im Bereich von 100 bis 280 °C und Drücken im Bereich von 50 bis 500 mbar abs. durchführt.

3. Verfahren nach Anspruch 1 oder 2,
dadurch gekennzeichnet,
daß man in die Trennwandkolonne sowie in die Destillationskolonne mit Vorkolonne oder Nachkolonne und/oder in die Vorkolonne oder in die Nachkolonne Strukturpackungen einbaut.

4. Verfahren nach mindestens einem der Ansprüche 1 bis 3,
dadurch gekennzeichnet,
daß man die p-TE-reiche Fraktion in die Oxidation zurückführt.

5. Verfahren nach mindestens einem der Ansprüche 1 bis 4,
dadurch gekennzeichnet,
daß man die schwersiedende Rückstandsfraktion einer Methanolyse oder Thermolyse sowie einer Betriebseinheit zur Katalysatorrückgewinnung zuführt.

6. Verfahren nach mindestens einem der Ansprüche 1 bis 5,
dadurch gekennzeichnet,
daß man die Roh-DMT-Fraktion zu Dimethylterephthalat mit höherer Reinheit und/oder Terephthalsäure weiterverarbeitet.

7. Verfahren zur Herstellung von Dimethylterephthalat (DMT), das im wesentlichen folgende Verfahrensschritte beinhaltet,
- Oxidation von para-Xylol (p-X) und para-Toluylsäuremethylester (p-TE),
- Veresterung der Reaktionsprodukte aus der Oxidation mit Methanol,
- Trennung des entstandenen, sogenannten Rohesters in eine p-TE-reiche Fraktion, eine Roh-DMT-Fraktion und eine schwersiedende Rückstandsfraktion,
- Reinigung der Roh-DMT-Fraktion,
nach mindestens einem der Ansprüche 1 bis 6,
dadurch gekennzeichnet,
daß man die Trennung des Rohesters in einer Trenneinheit durchführt, die mit einer Trennwandkolonne oder einer Destillationskolonne mit einer Vorkolonne oder einer Destillationskolonne mit einer Nachkolonne ausgestattet ist.
